# EUROPÄISCHE PATENTANMELDUNG

(11) **EP 3 392 326 A1**
(43) Veröffentlichungstag der Anmeldung: **24.10.2018**
(21) Anmeldenummer: 17166995.5
(22) Anmeldetag: 19.04.2017
(51) Int. Cl.: C10L 3/10, C07C 2/82

(54) **VERFAHREN UND ANLAGE ZUR HERSTELLUNG EINES OLEFINS**

(71) Anmelder: Linde AG, 80331 München (DE)
(72) Erfinder: Fritz, Helmut, 81375 München (DE); Oberle, David, 81476 München (DE); Mündl, Florian, 83624 Otterfing (DE); Stegemann, Robert, 81543 München (DE)
(74) Vertreter: Frommberger, Moritz

(57) **Zusammenfassung**

Es wird ein Verfahren (100) zur Herstellung eines Olefins vorgeschlagen, bei dem einer Reaktoreinheit (1) ein Methan enthaltender Reaktionseinsatz zugeführt wird, bei dem in der Reaktoreinheit (1) ein Anteil des Methans in dem Reaktionseinsatz durch oxidative Kopplung umgesetzt wird, und bei dem aus der Reaktoreinheit (1) ein Produktgemisch entnommen und von einem ersten Temperaturniveau auf ein zweites Temperaturniveau abgekühlt wird, wobei das Produktgemisch zumindest überwiegend in einer Hauptströmungsrichtung (10) aus der Reaktoreinheit (1) ausströmt. Es ist vorgesehen, dass für die Abkühlung eine Kühleinrichtung (2) verwendet wird, durch die das Produktgemisch zumindest überwiegend umlenkungsfrei und in der Hauptströmungsrichtung (10) geführt wird. Eine entsprechende Anlage ist ebenfalls Gegenstand der vorliegenden Erfindung.

## Beschreibung

Die Erfindung betrifft ein Verfahren zur Gewinnung eines Olefins und eine entsprechende Anlage gemäß den Oberbegriffen der jeweiligen unabhängigen Patentansprüche.

### Stand der Technik

Die oxidative Kopplung von Methan ist in der Literatur beschrieben, beispielsweise bei J.D. Idol et al., "Natural Gas", in: J.A. Kent (Hrsg.), "Handbook of Industrial Chemistry and Biotechnology", Band 2, 12. Auflage, Springer, New York 2012.

Die oxidative Kopplung von Methan umfasst nach derzeitigem Kenntnisstand eine katalysierte Gasphasenreaktion von Methan mit Sauerstoff, bei der von zwei Methanmolekülen jeweils ein Wasserstoffatom abgespalten wird. Die dabei entstehenden Methylradikale reagieren zunächst zu einem Ethanmolekül. Bei der Reaktion wird ferner ein Wassermolekül gebildet. Bei geeigneten Verhältnissen von Methan zu Sauerstoff, geeigneten Reaktionstemperaturen und der Wahl geeigneter Katalysebedingungen erfolgt anschließend eine Oxydehydrierung des Ethans zu Ethylen, einer Zielverbindung bei der oxidativen Kopplung von Methan. Hierbei wird ein weiteres Wassermolekül gebildet. Der eingesetzte Sauerstoff wird bei den genannten Reaktionen typischerweise vollständig umgesetzt.

Die Reaktionsbedingungen bei der oxidativen Kopplung von Methan umfassen klassischerweise eine Temperatur von 500 bis 900 °C, einen Druck von 5 bis 10 bar und hohe Raumgeschwindigkeiten. Jüngere Entwicklungen gehen insbesondere auch in Richtung der Verwendung tieferer Temperaturen. Die Reaktion kann homogen- und heterogenkatalytisch im Festbett oder in der Wirbelschicht erfolgen. Bei der oxidativen Kopplung von Methan können auch höhere Kohlenwasserstoffe mit bis zu sechs oder acht Kohlenstoffatomen gebildet werden, der Schwerpunkt liegt jedoch auf Ethan bzw. Ethylen und ggf. noch Propan bzw. Propylen.

Insbesondere aufgrund der hohen Bindungsenergie zwischen Kohlenstoff und Wasserstoff im Methanmolekül sind die Ausbeuten bei der oxidativen Kopplung von Methan vergleichsweise gering. Typischerweise werden nicht mehr als 10 bis 15% des eingesetzten Methans umgesetzt. Außerdem begünstigen die vergleichsweise harschen Reaktionsbedingungen und Temperaturen, die zur Spaltung dieser Bindungen erforderlich sind, auch die weitere Oxidation der Methylradikale und anderer Intermediate zu Kohlenmonoxid und Kohlendioxid.

Wenngleich den geringen Ausbeuten und der Bildung von Kohlenmonoxid und Kohlendioxid teilweise durch die Wahl optimierter Katalysatoren und angepasster Reaktionsbedingungen entgegengewirkt werden kann, enthält ein bei der oxidativen Kopplung von Methan gebildetes Gasgemisch neben den Zielverbindungen wie Ethylen und ggf. Propylen überwiegend nicht umgesetztes Methan sowie Kohlendioxid, Kohlenmonoxid und Wasser. Durch ggf. erfolgende nichtkatalytische Spaltreaktionen können auch beträchtliche Mengen an Wasserstoff enthalten sein. Ein derartiges Gasgemisch wird im hier verwendeten Sprachgebrauch auch als "Produktgemisch" der oxidativen Kopplung von Methan bezeichnet, obwohl es überwiegend nicht die gewünschten Produkte, sondern auch das nicht umgesetzte Edukt Methan und die soeben erläuterten Nebenprodukte enthält.

Bei der oxidativen Kopplung von Methan können Reaktoren eingesetzt werden, in denen einer katalytischen Zone eine nichtkatalytische Zone nachgeschaltet ist. Das aus der katalytischen Zone ausströmende Gasgemisch wird in die nichtkatalytische Zone überführt, wo es zunächst noch auf den vergleichsweise hohen Temperaturen vorliegt, die in der katalytischen Zone eingesetzt werden. Insbesondere durch die Anwesenheit des bei der oxidativen Kopplung von Methan gebildeten Wassers ähneln die Reaktionsbedingungen hier jenen herkömmlicher Dampfspaltverfahren (engl. Steam Cracking). Daher können hier Ethan und höhere Paraffine zu Olefinen umgesetzt werden. In die nichtkatalytische Zone können auch weitere Paraffine eingespeist werden, so dass die Restwärme der oxidativen Kopplung von Methan in besonders vorteilhafter Weise ausgenutzt werden kann. Ein derartiges gezieltes Dampfspalten in einer der katalytischen Zone nachgeschalteten nichtkatalytischen Zone wird auch als "Post Bed Cracking" bezeichnet. Nachfolgend wird hierfür auch der Begriff "postkatalytisches Dampfspalten" verwendet.

Bezüglich den beim Dampfspalten herrschenden Reaktionsbedingungen sei ebenfalls auf Fachliteratur wie den Artikel "Ethylene" in Ullmann's Encyclopedia of Industrial Chemistry, Onlineausgabe, 15. April 2007, DOI 10.1002/14356007.a10_045.pub2, verwiesen. Das Dampfspalten wird beispielsweise zur Gewinnung von kurzkettigen Olefinen wie Ethylen und Propylen, Diolefinen wie Butadien oder von Aromaten eingesetzt, ist jedoch nicht hierauf beschränkt.

Produktgemische, die unter Verwendung eines Verfahrens zur oxidativen Kopplung von Methan gebildet werden, enthalten aus den oben erläuterten Gründen insbesondere große Mengen an Methan, das vorteilhafterweise abgetrennt und in die oxidative Kopplung zurückgeführt werden sollte. Die trenntechnische Bearbeitung des Produktgemischs umfasst dabei herkömmlicherweise eine Kühlung auf Umgebungstemperatur. Hierbei kondensiert der überwiegende Teil des in dem Produktgemisch enthaltenen Wassers aus. Die Kühlung erfolgt auf dem Druck, auf dem das Produktgemisch stromab der oxidativen Kopplung von Methan vorliegt, also typischerweise 5 bis 10 bar, beispielsweise 7 bis 8 bar. Anschließend erfolgen eine Verdichtung und anschließend eine Entfernung des Kohlendioxids, beispielsweise unter Verwendung einer Amin- und/oder Laugenwäsche, aus dem Produktgemisch. Restwasser wird anschließend typischerweise adsorptiv, beispielsweise unter Verwendung von Molsiebadsorbern, abgetrennt.

Ein durch eine entsprechende Aufbereitung aus dem Produktgemisch erhaltenes Gasgemisch wird einer Tieftemperaturtrennung zugeführt, in der Methan und tiefer als Methan siedende Komponenten, also insbesondere Kohlenmonoxid und Wasserstoff, sofern enthalten, zumindest überwiegend abgetrennt werden. Das restliche Kohlenwasserstoffgemisch enthält überwiegend oder ausschließlich Ethylen und höher als Ethylen siedende Verbindungen, soweit in dem Produktgemisch enthalten. Hierbei sind unterschiedliche Ausgestaltungen (beispielsweise sogenannte "Demethanizer-First"- und "Deethanizer-First"-Verfahren) bekannt und beschrieben.

Bekannte Verfahren zur trenntechnischen Bearbeitung von Produktgemischen aus der oxidativen Kopplung von Methan arbeiten insbesondere hinsichtlich des erläuterten Abkühlschritts häufig unbefriedigend. Das Produktgemisch muss aber für einen möglichst effektiven Reaktionsumsatz einen möglichst schnellen Abkühlvorgang erfahren, da ansonsten die erwähnten unerwünschten Nebenreaktionen begünstigt und damit verstärkt Nebenprodukte gebildet würden.

Die vorliegende Erfindung will daher insbesondere die rasche Abkühlung eines Produktgemischs, das unter Einsatz der oxidativen Kopplung von Methan erhalten wurde, gegenüber dem Stand der Technik verbessern und energetisch und/oder apparativ optimieren.

### Offenbarung der Erfindung

Vor diesem Hintergrund schlägt die vorliegende Erfindung ein Verfahren zur Gewinnung eines Olefins und eine entsprechende Anlage gemäß den Oberbegriffen der jeweiligen unabhängigen Patentansprüche vor. Ausgestaltungen sind jeweils Gegenstand der abhängigen Patentansprüche sowie der nachfolgenden Beschreibung.

Vor der Erläuterung der Merkmale und Vorteile der vorliegenden Erfindung werden noch einige Grundlagen und die verwendeten Begriffe erläutert.

Ist nachfolgend davon die Rede, dass in der Reaktoreinheit ein Anteil des Methans in einem Reaktionseinsatz durch oxidative Kopplung umgesetzt wird, soll hierdurch explizit nicht ausgeschlossen sein, dass die Reaktoreinheit auch zur Durchführung weiterer Verfahren bzw. Verfahrensschritte eingerichtet sein kann, insbesondere des oben erläuterten postkatalytischen Dampfspaltens. Hierzu können auch separate Reaktoren vorgesehen sein. Entsprechenden weiteren Verfahren oder Verfahrensschritten können weitere Einsätze zugeführt werden, die zumindest teilweise zu Produkten umgesetzt werden, welche sich in einem der Reaktionseinheit entnommenen Produktgemisch wiederfinden. Unter einem "Produktgemisch" wird hierbei das der Reaktionseinheit entnommene Gasgemisch bezeichnet, obwohl dieses nicht nur das oder die gewünschten Produkte sondern ebenso nicht umgesetzte Edukte, insbesondere Methan, und Nebenprodukte enthält.

Flüssige und gasförmige Gemische können im hier verwendeten Sprachgebrauch reich oder arm an einer oder mehreren Komponenten sein, wobei "reich" für einen Gehalt von wenigstens 50%, 75%, 90%, 95%, 99%, 99,5%, 99,9% oder 99,99% und "arm" für einen Gehalt von höchstens 50%, 25%, 10%, 5%, 1%, 0,1% oder 0,01% auf molarer, Gewichts- oder Volumenbasis stehen kann. Der Begriff "überwiegend" kann der Definition von "reich" entsprechen.

Die vorliegende Anmeldung verwendet zur Charakterisierung von Drücken und Temperaturen die Begriffe "Druckniveau" und "Temperaturniveau", wodurch zum Ausdruck gebracht werden soll, dass entsprechende Drücke und Temperaturen in einer entsprechenden Anlage nicht in Form exakter Druck- bzw. Temperaturwerte verwendet werden müssen, um das erfinderische Konzept zu verwirklichen. Jedoch bewegen sich derartige Drücke und Temperaturen typischerweise in bestimmten Bereichen, deren Maximal- und Minimalwerte sich um beispielsweise nicht mehr als 1%, 5%, 10%, 20% oder sogar 50% unterscheiden.

Entsprechende Druckniveaus und Temperaturniveaus können dabei in disjunkten Bereichen liegen oder in Bereichen, die einander überlappen. Insbesondere schließen beispielsweise Druckniveaus unvermeidliche oder zu erwartende Druckverluste, beispielsweise aufgrund von Abkühlungseffekten, ein. Entsprechendes gilt für Temperaturniveaus. Bei den hier in bar angegebenen Druckniveaus handelt es sich jeweils um Absolutdrücke.

### Vorteile der Erfindung

Wie erwähnt, sind bekannte Verfahren zur trenntechnischen Bearbeitung von Produktgemischen aus der oxidativen Kopplung von Methan insbesondere hinsichtlich des erläuterten Abkühlschritts häufig unbefriedigend. Daher können gemäß dem Stand der Technik die erwähnten unerwünschten Nebenreaktionen häufig nicht in ausreichendem Maße unterdrückt werden, was sich nachteilig auf die Produktausbeuten in einem entsprechenden Verfahren auswirkt. Ein entsprechendes, zwischen 900 und 800 °C heißes Produktgemisch muss sehr schnell auf Temperaturen um 650 °C oder darunter (insbesondere weniger als 6 70 °C) abgekühlt werden um die unerwünschte Weiterreaktion der Zielproduktkomponenten (Ethylen und anderer Mono- und Diolefine) zu unterbinden.

Konventionelle Kühleinrichtungen wie beispielsweise Dampfkessel, die im U-Rohr-Design mit internen Bypässen oder im Geradrohrdesign ohne interne Bypässe ausgebildet sein können, erfordern beispielsweise eine 90°-Umlenkung des Prozessgases aus einem typischerweise vertikal von oben nach unten durchströmten Reaktor zur oxidativen Kopplung von Methan (Katalysatorbett mit anschließender nichtkatalytischer Zone). Bei dem Übergang zwischen Reaktor und Kühler kommt es daher herkömmlicherweise zu einer ungünstigen Strömungsbeeinflussung und damit zu Turbulenzen, Totzonen und einer Rückvermischung.

Die vorliegende Erfindung geht von einem Verfahren zur Herstellung eines Olefins aus, bei dem einer Reaktoreinheit ein Methan enthaltender Reaktionseinsatz zugeführt wird, bei dem in der Reaktoreinheit ein Anteil des Methans in dem Reaktionseinsatz durch oxidative Kopplung umgesetzt wird und bei dem aus der Reaktoreinheit ein Produktgemisch entnommen und von einem ersten Temperaturniveau auf ein zweites Temperaturniveau abgekühlt wird. Das Produktgemisch strömt im Rahmen der vorliegenden Erfindung aus der Reaktoreinheit zumindest überwiegend in einer Hauptströmungsrichtung aus.

Unter einer "Hauptströmungsrichtung" wird hier die mittlere Bewegungsrichtung aller aus der Reaktoreinheit ausströmenden Gasteilchen verstanden. Es versteht sich, dass nicht sämtliche Gasmoleküle eine völlig identische Bewegungsrichtung aufweisen können, sondern dass es auch bei einer in der Praxis niemals ideal auftretenden laminaren Strömung zumindest zu Randturbulenzen kommen kann. Die vorliegende Erfindung eignet sich für Festbett- und Wirbelschichtreaktoren. Insbesondere in letzteren können beträchtliche Turbulenzen auftreten.

Verfahren zur Abtrennung von Methan und tiefer siedenden Verbindungen aus unterschiedlichen Kohlenwasserstoffgemischen, beispielsweise aus Erdgas oder aus Gasgemischen, die unter Verwendung von Dampfspaltverfahren erhalten wurden, sind aus dem Stand der Technik grundsätzlich bekannt. Diese Verfahren weisen jedoch bei der trenntechnischen Bearbeitung von Gasgemischen, die unter Einsatz der oxidativen Kopplung von Methan erhalten wurden, Nachteile auf. Beispielsweise ist das für Erdgas verwendete sogenannte Recycle-Split-Vapor-(RSV-)Verfahren der Firma Ortloff, wie es in der US 4,157,904 A offenbart und bei H.-W. Häring (Hrsg.), Industrial Gases Processing, Wiley-VCH, 2006, Abschnitt 7.6.2, "Separation of Liquefied Petroleum Gas", näher erläutert ist, für die Trennung eines Gasgemischs, das unter Einsatz der oxidativen Kopplung von Methan erhalten wurde, nicht geeignet, da sich aufgrund der abweichenden Zusammensetzung die erforderlichen Reinheiten nicht ohne weiteres erzielen ließen. Der Fachmann hätte daher derartige Verfahren bei der trenntechnischen Bearbeitung eines Produktgemischs der oxidativen Kopplung von Methan nicht in Erwägung gezogen. Entsprechendes gilt auch beispielsweise für bekannte Verfahren zur trenntechnischen Bearbeitung von Produktgemischen aus Dampfspaltverfahren.

Im Rahmen der vorliegenden Erfindung ist vorgesehen, dass für die Abkühlung eine Kühleinrichtung verwendet wird, durch die das Produktgemisch zumindest überwiegend umlenkungsfrei und in der Hauptströmungsrichtung geführt wird. Auf diese Weise kann eine deutlich raschere Abkühlung als in einer herkömmlichen Kühleinrichtung wie beispielsweise einem Dampfkessel, bei dem eine entsprechende Umlenkung vorgesehen ist, erzielt werden. Bei dem Übergang zwischen Reaktoreinheit und Kühler kommt es im Rahmen der vorliegenden Erfindung nicht mehr zu einer ungünstigen Strömungsbeeinflussung und damit nicht oder kaum mehr zu Turbulenzen, Totzonen und einer Rückvermischung, und damit zu effektiv kürzeren Verweildauern und einem rascheren Durchlaufen eines Temperaturgradienten. In einer im Rahmen der vorliegenden Erfindung eingesetzten Kühleinrichtung ergeben sich aufgrund dieser Vorteile ferner auch deutlich geringere Verkokungseffekte als in herkömmlichen Verfahren.

Besonders vorteilhaft ist es, wenn in der Kühleinrichtung ein oder mehrere Wärmetauscher verwendet werden, der oder die eine Anzahl von Rohren umfassen, welche zumindest teilweise in der Hauptströmungsrichtung ausgerichtet sind und von dem Produktgemisch durchströmt werden. Im Rahmen der vorliegenden Erfindung kann auch die Reaktoreinheit mehrere Reaktoren umfassen, deren Produktgemisch jeweils einer Kühleinrichtung oder jeweils einem Wärmetauscher zugeführt wird. Es kann jedoch auch vorgesehen sein, das Produktgemisch mehrerer Reaktoreinheiten zusammenzufassen und dann einer gemeinsamen Kühleinrichtung oder einem gemeinsamen Wärmetauscher zuzuführen. Ebenso ist es möglich, dass das Produktgemisch eines Reaktors auf zwei oder mehrere Kühleinrichtungen oder deren Wärmetauscher aufgeteilt wird.

Vorteilhafterweise wird bei der Abkühlung des Produktgemischs Wärme zurückgewonnen. Hierzu kann bzw. können insbesondere ein oder mehrere Wärmetauscher verwendet werden, bei dem oder bei denen das Produktgemisch durch eine Anzahl Rohre geführt wird, die von einem Wasser- und/oder Dampfstrom umfströmt werden. Eine unter Verwendung eines derartigen Wärmetauschers erfolgende Dampferzeugung, Dampferwärmung oder Dampfüberhitzung kann zur Bereitstellung von Dampf in unterschiedlichen Dampfarten und Drücken verwendet werden. Beispielsweise kann im Rahmen der vorliegenden Erfindung unter Verwendung eines entsprechenden Wärmetauschers Sattdampf, überhitzter Dampf oder Hochdruckdampf erzeugt werden. Zu weiteren Details bezüglich entsprechender Dampfarten sei in diesem Zusammenhang auf einschlägige Fachliteratur zur Dampferzeugung verwiesen.

Besondere Vorteile werden erzielt, wenn in einem entsprechenden Wärmetauscher die Anzahl von Rohren vollständig in der Hauptströmungsrichtung ausgerichtet ist. Auf diese Weise kann eine vollständig ablenkungsfreie Abkühlung mit den zuvor erläuterten positiven Effekten erzielt werden. Insbesondere können in einem entsprechenden Wärmetauscher mehrere langgestreckte Rohre, die von dem Produktgemisch durchströmt werden, angeordnet sein und sich gerade oder im Wesentlichen gerade von einer Einlassplatte bis zu einer Endplatte erstrecken. Der grundsätzliche Aufbau eines entsprechenden Wärmetauschers ist beispielsweise in der EP 1 331 465 B1 gezeigt.

Insbesondere und mit besonderem Vorteil kann in einem entsprechenden Verfahren eine Kühleinrichtung mit einem Transferleitungswärmetauscher verwendet werden. Entsprechende Typen von Transferleitungswärmetauschern (engl. Transfer Line Heat Exchanger, TLE) sind dem Fachmann bekannt, beispielsweise aus der Fachliteratur zu Dampfspaltverfahren. Zu weiteren Details sei daher auf die entsprechende Fachliteratur, beispielsweise den erwähnten Artikel "Ethylene" in Ullmann's Encyclopedia of Industrial Chemistry, verwiesen. Dabei hätte der Fachmann aber Vorrichtungen und Verfahrensschritte aus Dampfspaltverfahren nicht ohne weiteres für die Bearbeitung von Produktgemischen aus der oxidativen Kopplung von Methan in Erwägung gezogen.

Im Rahmen des erfindungsgemäßen Verfahrens liegt das erste Temperaturniveau insbesondere bei 800 bis 900 °C und das zweite Temp eraturniveau bei 500 bis 700 °C. Das zweite Temperaturniveau liegt insbesondere bei höchstens 670 °C und weiter insbesondere bei höchstens 650 °C, weil sich bei de rartigen Temperaturen die erwähnten Weiterreaktionen im Wesentlichen zum Stillstand bringen lassen. Entsprechende Temperaturniveaus lassen sich mit den erwähnten Kühleinrichtungen bzw. Wärmetauschern erreichen. Insbesondere kann das zweite Temperaturniveau dabei durch die Länge und Anzahl der verwendeten Wärmeaustauscherrohre eingestellt werden. Die jeweils angestrebten zweiten Temperaturniveaus richten sich auch nach den betrieblichen Anforderungen und der Menge bzw. Temperatur des in einer entsprechenden Anlage zu erzeugenden Dampfs.

Das Verfahren der vorliegenden Erfindung kann mit unterschiedlichen Reaktorkonfigurationen eingesetzt werden. Insbesondere kann in derartigen Reaktorkonfigurationen die Hauptströmungsrichtung senkrecht aufwärts oder senkrecht abwärts gerichtet sein. Die Kühleinrichtung bzw. der oder die entsprechenden Wärmetauscher werden dann oberhalb oder unterhalb der Reaktoreinheit bzw. deren Reaktor oder Reaktoren angeordnet, so dass das Produktgemisch umlenkungsfrei durch die Kühleinrichtung mit dem oder den Wärmetauschern strömen kann.

Insbesondere kann die Reaktoreinheit wenigstens einen Festbettreaktor oder wenigstens einen Wirbelschichtreaktor umfassen, wie sie grundsätzlich aus dem Bereich der oxidativen Kopplung von Methan bekannt sind. Bei einem Festbettreaktor ist die Hauptströmungsrichtung dabei i.d.R. im Wesentlichen senkrecht abwärts gerichtet, bei einem Wirbelschichtreaktor hingegen senkrecht aufwärts, um die Wirbelschicht nicht aus dem Reaktor auszutragen.

Die vorliegende Erfindung erstreckt sich auch auf eine Anlage zur Herstellung eines Olefins, mit Mitteln, die dafür eingerichtet sind, einer Reaktoreinheit einen Methan enthaltenden Reaktionseinsatz zuzuführen, in der Reaktoreinheit einen Anteil des Methans in dem Reaktionseinsatz durch oxidative Kopplung umzusetzen, und aus der Reaktoreinheit ein Produktgemisch zu entnehmen und von dem ersten Temperaturniveau auf ein zweites Temperaturniveau abzukühlen, wobei die Reaktoreinheit derart ausgebildet ist, dass das Produktgemisch zumindest überwiegend in einer Hauptströmungsrichtung aus der Reaktoreinheit ausströmt.

Erfindungsgemäß ist für die Abkühlung eine Kühleinrichtung bereitgestellt, durch die das Produktgemisch zumindest überwiegend umlenkungsfrei und in der Hauptströmungsrichtung strömen kann. Auf die hierdurch erzielbaren Vorteile wurde bereits im Rahmen der Beschreibung des erfindungsgemäßen Verfahrens hingewiesen. Auf die entsprechenden Erläuterungen wird daher verwiesen.

Vorteilhafterweise umfasst die im Rahmen der vorliegenden Erfindung eingesetzte Reaktoreinheit wenigstens einen Reaktor, der einen Gaseinlass für den Reaktionseinsatz und einen Gasauslass für das Produktgemisch aufweist, wobei eine sich zwischen dem Gaseinlass und dem Gasauslass erstreckende geometrische Achse der Hauptströmungsrichtung entspricht. Ein entsprechender Reaktor kann auch eine im Wesentlichen zylindrische Hülle aufweisen, deren Zylinderachse der Hauptströmungsrichtung entspricht.

Vorteilhafterweise ist eine entsprechende Anlage zur Durchführung eines Verfahrens eingerichtet, wie es zuvor erläutert wurde, und weist hierzu jeweils entsprechende Mittel auf. Auch in diesem Zusammenhang wird auf die obigen Erläuterungen verwiesen.

Die Erfindung wird nachfolgend unter Bezugnahme auf die beigefügte Zeichnung näher erläutert, die eine Ausführungsform der Erfindung zeigt.

### Kurze Beschreibung der Zeichnung

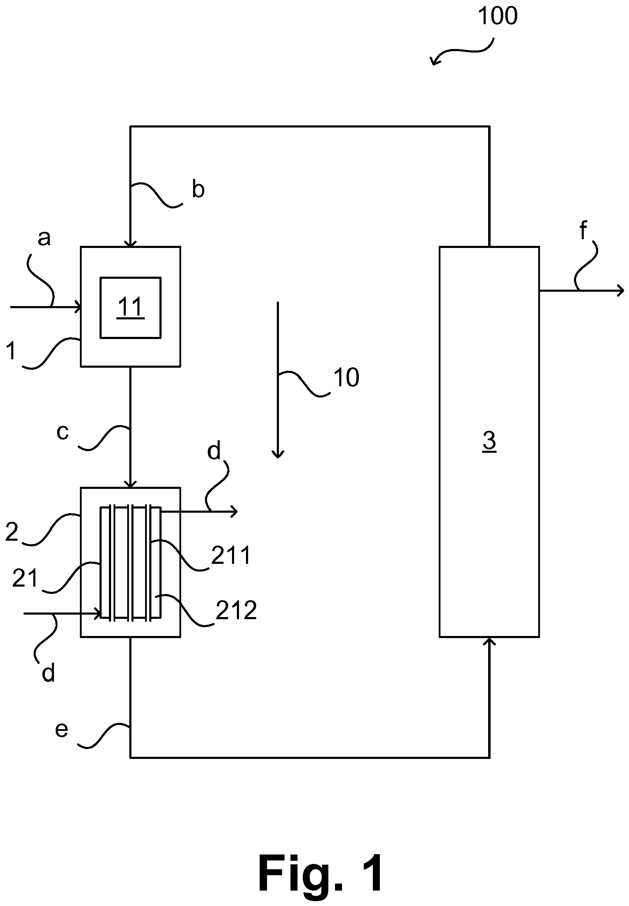

Figur 1 veranschaulicht ein Verfahren gemäß einer Ausführungsform der vorliegenden Erfindung anhand eines vereinfachten Anlagendiagramms.

### Ausführliche Beschreibung der Zeichnung

In Figur 1 ist ein Verfahren gemäß einer Ausführungsform der Erfindung anhand eines stark vereinfachten Anlagendiagramms veranschaulicht und insgesamt mit 100 bezeichnet. Die zu Figur 1 und dem Verfahren 100 erläuterten Komponenten bzw. Verfahrensschritte sind gleichzeitig Teil einer entsprechenden Anlage bzw. dort vorrichtungsmäßig implementiert, so dass die nachfolgenden Erläuterungen bezüglich Verfahrensschritten auch entsprechende Anlagen betreffen und umgekehrt. Die Darstellung ist insbesondere dahingehend stark vereinfacht, dass in der Praxis vorgesehene Vorrichtungen wie Regler, Ventile, für das Anfahren einer entsprechenden Anlage erforderliche Bypässe usw. nicht veranschaulicht sind. Der Fachmann sieht entsprechende Elemente nach Bedarf vor. Die Darstellung ist nicht maßstabsgetreu.

In dem Verfahren 100 wird eine oxidative Kopplung von Methan in einer Reaktionseinheit 1 durchgeführt, die einen oder mehrere Reaktoren 11 umfassen kann, wie hier in sehr vereinfachter Form gezeigt. Der oxidativen Kopplung von Methan können weitere Verfahren bzw. Verfahrensschritte zu- bzw. nachgeordnet sein, wie einleitend erläutert und nicht gesondert dargestellt.

Die Reaktionseinheit 1 kann insbesondere mit einem oder mehreren von extern zugeführten methanreichen Stoffströmen a sowie mit einem oder mehreren aus den Verfahren 100 rückgeführten methanreichen Stoffströmen b gespeist werden. Der Reaktionseinheit 1 können ferner ein oder mehrere sauerstoffreiche Stoffströme zugeführt werden, wie hier nicht gesondert dargestellt. Wird ein eingangs erläutertes postkatalytisches Dampfspalten eingesetzt, können diesem ein oder mehrere paraffinreiche Stoffströme, beispielsweise ein oder mehrere Ethan- oder Propanströme, zugeführt werden. Auch diese können in den Verfahren 100 oder stromab hiervon gebildet oder von extern zugeführt werden. Sämtliche dieser eingesetzten Gase, Gasgemische, Stoffströme usw. werden im Rahmen dieser Anmeldung als "Reaktionseinsatz" zusammengefasst.

Unter Einsatz der oxidativen Kopplung von Methan wird durch die Reaktionseinheit 1, d.h. auch ggf. unter Einsatz weiterer Verfahren bzw. Verfahrensschritte, ein Gasgemisch bereitgestellt, das neben Methan tiefer als Methan siedende Komponenten, insbesondere Wasserstoff und Kohlenmonoxid, sowie höher als Methan siedende Kohlenwasserstoffe enthält. Hierbei wird ein Anteil des Methans in dem Reaktionseinsatz durch oxidative Kopplung umgesetzt. Dieses Gasgemisch, hier, wie erwähnt, als "Produktgemisch" bezeichnet, wird in Form eines Stoffstroms c aus der Reaktionseinheit 1 ausgeführt. Das Produktgemisch strömt dabei überwiegend in einer Hauptströmungsrichtung aus der Reaktoreinheit 1 aus. Die Hauptströmungsrichtung ist im dargestellten Beispiel, das sich insbesondere auf einen Festbettreaktor in der Reaktoreinheit 1 bezieht, senkrecht nach unten gerichtet und mit einem Pfeil 10 veranschaulicht. Wie erwähnt, kann die Hauptströmungsrichtung aber auch entgegengesetzt hierzu gerichtet sein, insbesondere bei einem Wirbelschichtreaktor. Stromab der Reaktoreinheit 1, und im dargestellten Beispiel aufgrund der vorliegenden Hauptströmungsrichtung unterhalb dieser, ist eine Kühleinrichtung 2 bereitgestellt, durch die das Produktgemisch zumindest überwiegend umlenkungsfrei und in der Hauptströmungsrichtung geführt wird. In dem in Figur 1 veranschaulichten Beispiel des Verfahrens 100 umfasst die Kühleinrichtung 2 einen Wärmetauscher 21, der eine Anzahl von Rohren 211 aufweist, welche im dargestellten Beispiel vollständig entlang der Hauptströmungsrichtung ausgerichtet sind und von dem Produktgemisch durchströmt werden. In Figur 1 ist nur eine sehr reduzierte Anzahl von Rohren 211 veranschaulicht. Die Anzahl von Rohren 211 ist beispelsweise in einem Mantelraum 212 angeordnet, welcher von einem Wasser- und/oder Dampfstrom, hier mit d bezeichnet, durchströmt wird. Alternativ wird der Wasser- und/oder Dampfstrom in Außenrohren geführt, in welchen die Rohre 211 konzentrisch angeordnet sind.

Nachdem das Produktgemisch in der Kühleinrichtung 2 von einem ersten auf ein zweites Temperaturniveau abgekühlt wurde, wird es in Form eines Stoffstroms e weiteren Verfahrensschritten zugeführt, die hier insgesamt mit 3 zusammengefasst sind und nicht im Detail veranschaulicht sind. Diese umfassen beispielsweise eine Verdichtung, eine Kohlendioxidwäsche, eine Trocknung, eine Kühlung und eine oder mehrere kryogene Trennschritte. In letzteren kann beispielsweise der in die Reaktoreinheit 1 zurückgeführte, methanreiche Stoffstrom b und ein Produktstrom f, der aus dem Verfahren ausgeführt wird, gewonnen werden.

## Patentansprüche

1. Verfahren (100) zur Herstellung eines Olefins, bei dem einer Reaktoreinheit (1) ein Methan enthaltender Reaktionseinsatz zugeführt wird, bei dem in der Reaktoreinheit (1) ein Anteil des Methans in dem Reaktionseinsatz durch oxidative Kopplung umgesetzt wird, und bei dem aus der Reaktoreinheit (1) ein Produktgemisch entnommen und von einem ersten Temperaturniveau auf ein zweites Temperaturniveau abgekühlt wird, wobei das Produktgemisch zumindest überwiegend in einer Hauptströmungsrichtung (10) aus der Reaktoreinheit (1) ausströmt, **dadurch gekennzeichnet, dass** für die Abkühlung eine Kühleinrichtung (2) verwendet wird, durch die das Produktgemisch zumindest überwiegend umlenkungsfrei und in der Hauptströmungsrichtung (10) geführt wird.

2. Verfahren (100) nach Anspruch 1, bei dem die Kühleinrichtung (2) einen Wärmetauscher (21) umfasst, der eine Anzahl von Rohren (211) umfasst, welche zumindest teilweise in der Hauptströmungsrichtung (10) ausgerichtet sind und von dem Produktgemisch durchströmt werden.

3. Verfahren (100) nach Anspruch 2, bei dem die Anzahl von Rohren in dem Wärmetauscher (21) zumindest teilweise von einem Wasser- und/oder Dampfstrom umströmt werden.

4. Verfahren (100) nach Anspruch 2 oder 3, bei dem die Anzahl von Rohren vollständig in der Hauptströmungsrichtung (10) ausgerichtet ist.

5. Verfahren (100) nach einem der vorstehenden Ansprüche, bei dem eine Kühleinrichtung (21) mit einem Transferleitungswärmetauscher verwendet wird.

6. Verfahren (100) nach einem der vorstehenden Ansprüche, bei dem das erste Temperaturniveau bei 800 bis 900 °C liegt und bei dem das zweite Temperaturniveau bei 500 bis 700 °C liegt.

7. Verfahren (100) nach einem der vorstehenden Ansprüche, bei dem die Hauptströmungsrichtung (10) entweder überwiegend senkrecht aufwärts oder senkrecht abwärts gerichtet ist.

8. Verfahren (100) nach einem der vorstehenden Ansprüche, bei dem die Reaktoreinheit (1) wenigstens einen Festbettreaktor oder wenigstens einen Wirbelschichtreaktor umfasst.

9. Anlage zur Herstellung eines Olefins, mit Mitteln, die dafür eingerichtet sind, einer Reaktoreinheit (1) einen Methan enthaltenden Reaktionseinsatz zuzuführen, in der Reaktoreinheit (1) einen Anteil des Methans in dem Reaktionseinsatz durch oxidative Kopplung umzusetzen, und aus der Reaktoreinheit (1) ein Produktgemisch zu entnehmen und von dem ersten Temperaturniveau auf ein zweites Temperaturniveau abzukühlen, wobei die Reaktoreinheit (1) derart ausgebildet ist, dass das Produktgemisch zumindest überwiegend in einer Hauptströmungsrichtung (10) aus der Reaktoreinheit (1) ausströmt, **dadurch gekennzeichnet, dass** für die Abkühlung eine Kühleinrichtung (2) bereitgestellt ist, durch die das Produktgemisch zumindest überwiegend umlenkungsfrei und in der Hauptströmungsrichtung (10) strömen kann.

10. Anlage nach Anspruch 9, bei dem die Reaktoreinheit (1) wenigstens einen Reaktor umfasst, der einen Gaseinlass für den Reaktionseinsatz und einen Gasauslass für das Produktgemisch aufweist, wobei eine sich zwischen dem Gaseinlass und dem Gasauslass erstreckende geometrische Achse der Hauptströmungsrichtung (10) entspricht.

11. Anlage nach Anspruch 9 oder 10, die zur Durchführung eines Verfahrens nach einem der Ansprüche 1 bis 8 eingerichtet ist.
